# EUROPÄISCHE PATENTSCHRIFT

(11) **EP 0 369 208 B1**
(45) Veröffentlichungstag und Bekanntmachung des Hinweises auf die Patenterteilung: **17.03.1993**
(21) Anmeldenummer: 89119640.4
(22) Anmeldetag: 23.10.1989
(51) Int. Cl.: C07D 213/68

(54) **Verfahren zur Herstellung von 3,5-Dimethyl-4-methoxypyridinderivaten sowie neues Zwischenprodukt hierfür**
Process for the preparation of 3,5-dimethyl-4-methoxy pyridine derivatives, and intermediate therefor
Procédé de préparation de dérivés de la diméthyl-3,5 méthoxy-4 pyridine et produits intermédiaires à cette fin

(30) Priorität: 15.11.1988 AT 2789/88
(43) Veröffentlichungstag der Anmeldung: 23.05.1990
(73) Patentinhaber: Hafslund Nycomed Pharma Aktiengesellschaft, A-4021 Linz (AT)
(72) Erfinder: Baumann, Karl, Dr. Dipl.-Ing., A-1190 Wien (AT)
(74) Vertreter: Kunz, Ekkehard, Dr.

(56) Entgegenhaltungen:
- EP-A- 0 103 553
- EP-A- 0 226 558
- US-A- 4 159 382

## Beschreibung

Die Erfindung betrifft ein neues Verfahren zur Herstellung von 3,5-Dimethyl-4-methoxypyridin-2-methanol bzw. von 3,5-Dimethyl-4-methoxy-2-chlormethylpyridin, sowie das bei diesem Verfahren entstehende neue Zwischenprodukt 3,5-Dimethyl-4-methoxypyridin-2-methanamin und dessen Verwendung.

3,5-Dimethyl-4-methoxypyridin-derivate stellen wertvolle Zwischenprodukte bei der Herstellung von ATP-asehemmern wie sie etwa in der EP 0 005 129 beschrieben sind, dar.

Aus EP-A-0 080 602 ist die Herstellung von 3,5-Dimethyl-4-methoxypyridin-2-methanol bzw. von 3,5-Dimethyl-4-methoxy-2-chlormethylpyridin ausgehend von 2,3,5-Trimethylpyridin bekannt, wobei diese Verbindung vorerst mit H₂O₂ zum N-oxid umgesetzt wird, worauf dieses N-oxid zum 2,3,5-Trimethyl-4-nitropyridin-N-oxid nitriert und die NO₂-Gruppe durch Behandeln mit Natriummethoxid gegen eine Methoxygruppe ausgetauscht wird. Das entstandene 2,3,5-Trimethyl-4-methoxypyridin-N-oxid wird anschließend mit Essigsäureanhydrid versetzt, das entstandene Acetylderivat zum 3,5-Dimethyl-4-methoxy-pyridin-2-methanol verseift und gegebenenfalls mit Thionylchlorid zu 3,5-Dimethyl-4-methoxy-2-chlormethylpyridin umgesetzt.

Aus EP-A-0 103 553 ist ein weiteres Verfahren bekannt, bei dem durch Umsetzung des wie in der EP-A-0 080 602 beschrieben hergestellten 3,5-Dimethyl-4-methoxypyridin-N-oxids mit Dimethylsulfat vorerst 1,4-Dimethoxy-3,5-Dimethylpyridinium-methylsulfat und durch anschließende Umlagerung 3,5-Dimethyl-4-methoxypyridin-2-methanol erhalten wird.

Die Ausgangsverbindung für diese Verfahren, das 2,3,5-Trimethylpyridin ist nur schwer und in schlechter Ausbeute zugänglich, etwa durch Reaktion von 3,5-Dimethylpyridin mit Methyllithium bei tiefen Temperaturen.

In der EP-A-0 226 558 wird die Herstellung der Titelverbindungen ausgehend von 2-Methylacetessigsäureethylester durch Reaktion mit NH₃ zu Ethyl-3-amino-2- methylcrotonat, Umsetzung mit Diethyl(monomethyl)malonat zum 2,3,5-Trimethyl-4,6-dihydroxypyridin, Chlorierung zu 2,4-Dichlor-3,5,6-trimethylpyridin, selektive Abspaltung des 2-Cl-Substituenten und Austausch des 4-Cl-Substituenten gegen eine Methoxygruppe, sowie anschließende Oxidation zum N-oxid beschrieben. Die weiteren Umsetzungen erfolgen analog dem in der EP-A-0 080 602 beschriebenen Verfahren.

Bei diesem Verfahren muß das für die Umlagerungen zur Modifizierung der in 2-Stellung gebundenen Methylgruppe benötigte 2,3,5-Trimethyl-4-Methoxypyridin-N-oxid in einem komplizierten 7-stufigen Verfahren aus aliphatischen Verbindungen hergestellt werden.

Es konnte nun ein einfaches Verfahren zur Herstellung von 3,5-Dimethyl-4-methoxypyridin-2-methanol bzw. 3,5-Dimethyl-4-methoxy-2-chlormethylpyridin ausgehend von 3,5-Dimethyl-4-methoxypyridin-2-carbonitril, das in Chemical Abstracts, Vol 106, 102092f (1987) beschrieben ist, gefunden werden.

Gegenstand der Erfindung ist demnach ein Verfahren zur Herstellung von Verbindungen der allgemeinen Formel 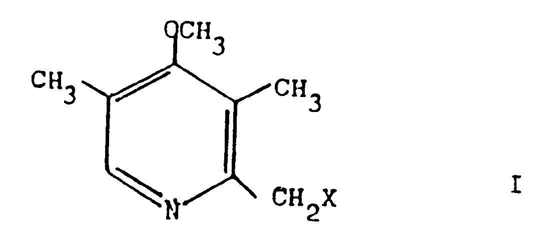
in der X die Reste OH oder Cl bedeutet, das dadurch gekennzeichnet ist, daß man 3,5-Dimethyl-4-methoxypyridin-2-carbonitril der Formel 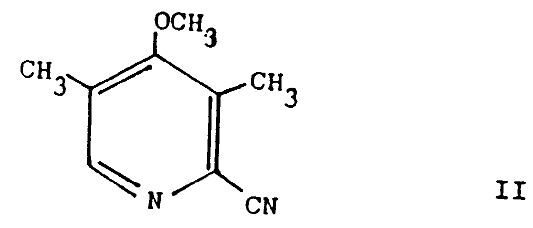
in Gegenwart eines inerten Verdünnungsmittels katalytisch hydriert, anschließend das entstandene 3,5-Dimethyl-4-methoxypyridin-2-methanamin der Formel 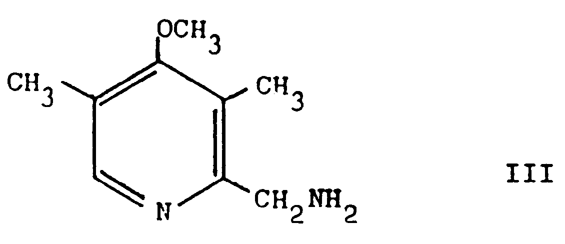
mit Natriumnitrit in wäßriger saurer Lösung zu 3,5-Dimethyl-4-methoxypyridin-2-methanol umsetzt und gegebenenfalls anschließend mit Thionylchlorid zu 3,5-Dimethyl-4-methoxy-2-chlormethylpyridin umsetzt.

Das bei diesem Verfahren entstehende Zwischenprodukt 3,5-Dimethyl-4-methoxypyridin-2-methanamin ist neu und ein weiterer Gegenstand der Erfindung.

Das Ausgangsprodukt für dieses Verfahren, 3,5-Dimethyl-4-methoxypyridin-2-carbonitril, kann auf einfache Weise ausgehend von 3,5-Dimethylpyridin durch Oxidation zum 3,5-Dimethylpyridin-N-oxid, Nitrierung und Umsetzung mit Dimethylsulfat zum 1,4-Dimethoxy-3,5-dimethylpyridinium-methylsulfat Behandlung mit KCN und anschließenden Austausch der Nitrogruppe gegen eine Methoxygruppe mit Natriummethoxid hergestellt werden.

In einem ersten Schritt erfolgt die Hydrierung der Carbonitrilgruppe zur Methylamingruppe. Die Hydrierung wird in Gegenwart eines Hydrierkatalysators und eines unter Reaktionsbedingungen inerten Verdünnungsmittel durchgeführt.
Als Katalysatoren können beispielsweise Raney-Nickel oder Pd auf Aktivkohle verwendet werden. Zur Verhinderung der Bildung des sekundären Amins wird vorzugsweise eine Base, wie NH₃ oder eine Säure, beispielsweise eine Mineralsäure oder Toluolsulfonsäure zugegeben, wobei bei Zusatz einer Säure bis zu 2 Äquivalenten eingesetzt werden.

Bevorzugt wird Raney-Nickel in Verbindung mit NH₃, oder Pd/C in Verbindung mit einer Säure, besonders bevorzugt Raney-Nickel mit NH₃ verwendet.
Als unter Reaktionsbedingungen inerte Verdünnungsmittel kommen beispielsweise Alkohole, wie Methanol oder Ethanol, Ether, wie Dioxan, Tetrahydrofuran oder Mischungen der Verdünnungsmittel mit Wasser in Betracht. Bevorzugt wird Methanol verwendet. Die Hydrierung erfolgt abhängig von den übrigen Reaktionsbedingungen bei etwa 20 - 100°C, vorzugsweise bei Raumtemperatur, und unter einem Druck von etwa 1 bis 5 bar, vorzugsweise bei Normaldruck.

Zur Isolierung des bei der Hydrierung entstandenen Zwischenproduktes 3,5-Dimethyl-4-methoxypyridin-2-methanamin wird nach beendeter Reaktion der Katalysator abfiltriert und das Lösungsmittel entfernt. Der verbleibende Rückstand kann durch übliche Methoden, beispielsweise durch Vakuum-Destillation weiter gereinigt und kristallisiert werden.

Die Umsetzung des neuen Zwischenproduktes zu 3,5-Dimethyl-4-methoxypyridin-2-methanol erfolgt durch Diazotierung der Aminogruppe unter hydrolysierenden Bedingungen.
Die Reaktion wird in wäßriger saurer Lösung durchgeführt. Als Säuren kommen Mineralsäuren oder Essigsäure in Betracht. Vorzugsweise wird Eisessig als Säure verwendet. Das Verhältnis von Wasser zu Säure kann in einem weiten Bereich schwanken, vorzugsweise beträgt es etwa 9 : 1 bis 1 : 1. Die Diazotierung wird mit Natriumnitrit durchgeführt, wobei Natriumnitrit in fester Form oder in Lösung eingesetzt werden kann. Die Umsetzung erfolgt bei Temperaturen von etwa -20 bis 50°C, vorzugsweise bei etwa -5 bis 10°C.
Nach beendeter Reaktion wird die wäßrige Lösung alkalisiert und das Produkt mit einem organischen Lösungsmittel, beispielsweise Methylenchlorid extrahiert und die organische Phase getrocknet.
Zur Isolierung wird die organische Phase filtriert und das Produkt beispielsweise durch Kugelrohrdestillation gereinigt.

Gegebenenfalls wird das so hergestellte 3,5-Dimethyl-4-methoxypyridin-2-methanol anschließend mit Thionylchlorid zum 3,5-Dimethyl-4-methoxy-2-chlormethylpyridin umgesetzt.

Die Reaktion erfolgt in Gegenwart eines unter Reaktionsbedingungen inerten Verdünnungsmittels, beispielsweise in Dichlorethan, Methylenchlorid, Chloroform, Dioxan oder Tetrahydrofuran.
Je nach den übrigen Reaktionsbedingungen erfolgt die Umsetzung bei etwa -30 bis 50°C, vorzugsweise bei etwa -20 bis 0°C.
Nach beendeter Reaktion wird das Verdünnungsmittel verdampft, der Rückstand beispielsweise in i-Propanol digeriert und 3,5-Dimethyl-4-methoxy-2-chlormethylpyridin-hydrochlorid isoliert.

Vorzugsweise wird das Verfahren ausgehend von 3,5-Dimethyl-4-methoxypyridin-2-carbonitril jedoch ohne Isolierung der Zwischenprodukte bis zum 3,5-Dimethyl-4-methoxy-2-chlormethylpyridin durchgeführt.

Dabei wird nach der Hydrierung der Katalysator abfiltriert und das Verdünnungsmittel entfernt. Der Rückstand wird daraufhin in saurer wäßriger Lösung aufgenommen und die Diazotierung unter hydrolysierenden Bedingungen durchgeführt.

Das nach der Extraktion in einem organischen Lösungsmittel vorliegende 3,5-Dimethyl-3-methoxypyridin-2-methanol wird dann mit Thionylchlorid zum 3,5-Dimethyl-4-methoxy-2-chlormethylpyridin umgesetzt und wie oben beschrieben isoliert.

Das Verfahren verläuft in guter Gesamtausbeute. Im allgemeinen werden Ausbeuten von 40 - 50 % bezogen auf 3,5-Dimethyl-4-methoxypyridin-2-carbonitril erreicht.

### Beispiel 1:

### 3,5-Dimethyl-4-methoxypyridin-2-methanamin

Eine Mischung aus 20 g Raney-Nickel und 20 g 3,5-Dimethyl-4-methoxypyridin-2-carbonitril in 650 ml NH₃ gesättigtem Methanol, hergestellt durch Einleiten von trockenem NH₃-Gas, wurde 3 Tage bei Raumtemperatur und Normaldruck hydriert. Anschließend wurde mit Ar gespült, der Katalysator abfiltriert und das Lösungsmittel am Rotationsverdampfer entfernt. Der zurückbleibende, violett gefärbte flüssige Rückstand wurde durch Kugelrohrdestillation gereinigt.
T: 120°C Luftbadtemperatur, p 0,05 bar.
Es wurden 13,5 g (65,9 % d. Th.) der Titelverbindung als farbloses Öl erhalten.
1H NMR (300 MHZ/TMS)
(ppm) = 1,94 (sb, 2H, -NH₂), 2,21 (s, 3H, -CH₃) 2,24 (s, 3H, -CH₃), 3,75 (s, 3H, -OCH₃), 3,90 (s, 2H, -CH₂), 8,21 (s, 1H, Pyr-H)

### Beispiel 2:

### 3,5-Dimethyl-4-methoxypyridin-2-methanol

Eine Lösung von 2,9 g 3,5-Dimethyl-4-methoxypyridin-2-methanamin in 50 ml 10 %iger wäßriger Essigsäure wurde bei 0°C portionsweise mit einer Lösung von 3,61 g NaNO₂ in 20 ml H₂O versetzt und 1 h nachgerührt. Anschließend wurde zwischen 30 ml 4 n NaOH und 50 ml CH₂Cl₂ verteilt die organische Phase abgetrennt und die wäßrige Phase noch 3 mal mit je 50 ml CH₂Cl₂ extrahiert. Die vereinigten organischen Phasen wurden über Na₂SO₄ getrocknet, filtriert und eingedampft. Das kristalline braune Rohprodukt wurde durch Kugelrohrdestillation gereinigt.
T: 115 - 135°C Luftbadtemperatur, p: 0,01 bar
Es wurden 2,57 g (88,3 % d.Th.) der Titelverbindung als farbloses Öl erhalten, das bei Raumtemperatur erstarrt.
1H NMR(300 MHZ/TMS/CDCl₃)
(ppm) = 2,12 (s, 3H, CH₃), 2,26 (s, 3H, CH₃), 3,77 (s, 3H, OCH₃), 4,62 (2, 2H, -CH₂-), 4,9 (sb, 1H, -OH), 8,19 (s, 1H, Pyr-H)

### Beispiel 3:

### Herstellung der Ausgansverbindung 3,5-Dimethyl-4-methoxypyridin-2-carbonitril

a) 3,5-Dimethylpyridin-N-oxid
   Zu 350 g 3,5-Dimethylpyridin wurden 98 ml Eisessig bei 25°C zugetropft. Die Mischung wurde auf 50°C erwärmt und 870,3 g 40 %ige Peressigsäure so zudosiert, daß die Temperatur durch Kühlung auf 50°C gehalten wurde. Etwa 1 Stunde nach beendeter Peressigsäurezugabe wurde die Reaktionsmischung auf 25°C gekühlt und 230 g Na₂SO₃ in 1 l Wasser unter Kühlung zugetropft. Nach Zerstörung des Überschusses an Peressigsäure (KJ-Stärke-Test) wurden 1330 ml Wasser zugegeben, mit 730 ml 50 %iger NaOH alkalisiert und die Lösung mit insgesamt etwa 4500 ml Chloroform in mehreren Portionen extrahiert. Die vereinigten organischen Phasen wurden mit Na₂SO₄ getrocknet und das Lösungsmittel bis auf etwa 740 ml abgedampft.
b) 3,5-Dimethyl-4-nitropyridin-N-oxid
   Zu 577,5 ml konzentrierter Schwefelsäure wurde bei einer Temperatur von 40°C und einem Druck von etwa 0,26 bar die aus a) erhaltene Lösung unter gleichzeitigem Abziehen des Lösungsmittel zugetropft. Anschließend wurde die Lösung unter Wasserstrahlvakuum 1 h auf 45°C gehalten, dann auf 70°C erwärmt und 577,5 ml rauchende HNO₃ unter Kühlung so zugetropft, daß die Temperatur 80 - 85°C nicht überstieg. Die Mischung wurde über Nacht abgekühlt und auf eine Mischung aus 3 kg Eis und 2 l Wasser unter Kühlung zugetropft. Anschließend wurde mit etwa 720 ml 50 %iger NaOH alkalisiert und mit etwa 9 l Chloroform in mehreren Portionen extrahiert. Die vereinigten organischen Phasen wurden mit Na₂SO₄ getrocknet, auf etwa 900 ml eingeengt bei Raumtemperatur mit 500 ml Diisopropylether versetzt und der entstandene Niederschlag abfiltriert und getrocknet. Es wurden 427 g (77,7 % d. Th) der Titelverbindung erhalten. Fp.: 180 - 183°C (subl.)
c) 1,4-Dimethoxy-3,5-dimethylpyridinium-methylsulfat
   4050 ml Ethylacetat, 400 g des Produktes aus b) und 390 g Dimethylsulfat wurden 4 h unter Rückfluß erhitzt, über Nacht abgekühlt und mit etwa 1600 ml Wasser in mehreren Portionen extrahiert.
d) 3,5-Dimethyl-4-nitropyridin-2-carbonitril
   464,9 g KCN wurden in 3500 ml Wasser gelöst, auf 5°C abgekühlt und 1600 ml der wäßrigen Lösung aus c) unter Kühlung zugetropft. Etwa 1 h nach Ende des Zutropfens wurde der Niederschlag filtriert, mit Wasser gewaschen und über P₂O₅ getrocknet. Es wurden 377,4 g der Titelverbindung (89,3 % d. Th. bezogen auf b) erhalten. Fp.: 66 - 71°C
   1 H NMR (300 MHZ/TMS/CDCl₃)
   (ppm)= 2,40 (s, 3H, CH₃), 2,53 (s, 3H, CH₃), 8,59 (s, 1H, Pyr-H)
e) 3,5-Dimethyl-4-methoxypyridin-2-carbonitril
   350 g des Produktes aus d) wurden in 2100 ml Methanol gelöst auf Rückflußtemperatur erhitzt und eine Lösung von 391 g NaOCH₃ in 1500 ml Methanol zugetropft. Nach Ende der Reaktion wurden 10 g Aktivkohle zugesetzt, auf 30°C abgekühlt, filtriert, die Lösung in Vakuum auf etwa 1,5 l eingeengt. Das Gemisch wurde auf 7,5 l Eiswasser geleert, der entstandene Niederschlag wurde abgesaugt mit Wasser gewaschen und über P₂O₅ getrocknet.
   Es wurden 288,4 g der Titelverbindung (90 % d. Th. bezogen auf d) erhalten.
   Fp.: 59 - 61°C
   1H NMR (300 MHZ /TMS/CDCl₃)
   (ppm)= 2,34 (s, 3H, CH₃), 2,47 (s, 3H, CH₃), 3,84 (s, 3H, OCH₃), 8,32 (s, 1H, Pyr-H)

### Beispiel 4:

### 3,5-Dimethyl-4-methoxy-2-chlormethylpyridin-hydrochlorid

20 g 3,5-Dimethyl-4-methoxypyridin-2-carbonnitril wurden in 650 ml Methanol gesättigt mit NH₃ gelöst, 20 g Raney-Nickel unter N₂-Atmosphäre zugegeben und hydriert. Der Katalysator wurde anschließend abfiltriert und die Lösung in Vakuum eingedampft.
Der Rückstand, 21,3 g violettes Öl wurde in 100 ml Wasser und 100 ml Eisessig gelöst, auf 0°C gekühlt und unter Kühlen 25,4 g NaNO₂ in 50 ml Wasser zugetropft. Anschließend wurde mit 500 ml 4n NaOH und etwa 30 ml 50 %iger NaOH alkalisiert, mit 600 ml CH₂Cl₂ extrahiert, und die vereinigten organischen Phasen über Na₂SO₄ getrocknet.
Die getrocknete CH₂Cl₂-Lösung wurde auf -10°C abgekühlt und 50,11 g SOCl₂ zugetropft. Anschließend wurde die Lösung im Vakuum bei 45°C eingedampft, der Rückstand mit i-Propanol verrührt und abgesaugt.

Es wurden 11,25 g (41,07 % d. Th) der Titelverbindung erhalten.
1H NMR (300 MHZ/TMS/CDCl₃)
(ppm)= 2,46 (s, 3H, CH₃), 2,48 (s, 3H, CH₃), 4,09 (s, 3H, OCH₃), 5,12 (s, 2H, CH₂), 8,39 (s, 1H, Pyr-H)

## Patentansprüche (Patentansprüche für folgende(n) Vertragsstaat(en): AT, BE, CH, DE, FR, GB, IT, LI, LU, NL, SE)

1. Verfahren zur Herstellung von Verbindungen der allgemeinen Formel 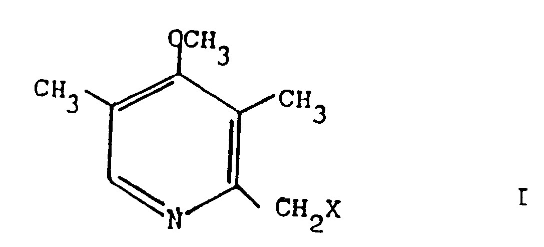 in der X die Reste OH oder Cl bedeutet, dadurch gekennzeichnet, daß man 3,5-Dimethyl-4-methoxypyridin-2-carbonitril der Formel 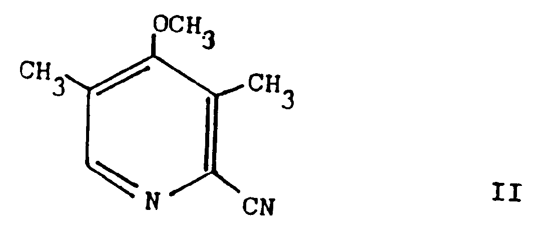 in Gegenwart eines inerten Verdünnungsmittels katalytisch hydriert, anschließend das entstandene 3,5-Dimethyl-4-methoxypyridin-2-methanamin der Formel 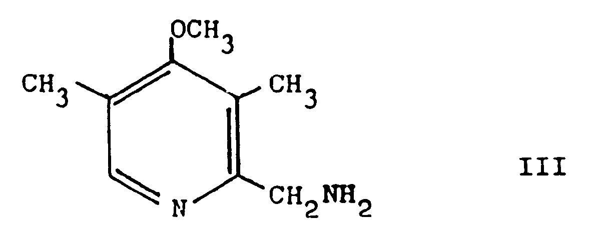 mit Natriumnitrit in wäßriger saurer Lösung zu 3,5-Dimethyl-4-methoxypyridin-2-methanol umsetzt und gegebenenfalls anschließend mit Thionylchlorid zu 3,5-Dimethyl-4-methoxy-2-chlormethylpyridin umsetzt.

2. Verfahren nach Anspruch 1, dadurch gekennzeichnet, daß die katalytische Hydrierung mit Raney-Nickel oder Pd auf Aktivkohle als Katalysator durchgeführt wird.

3. Verfahren nach einem der Ansprüche 1 oder 2, dadurch gekennzeichnet, daß die katalytische Hydrierung mit Raney-Nickel in Verbindung mit NH₃ oder mit Pd auf Aktivkohle in Verbindung mit Mineralsäuren oder Toluolsulfonsäure durchgeführt wird.

4. Verfahren nach einem der Ansprüche 1 bis 3, dadurch gekennzeichnet, daß die katalytische Hydrierung mit Raney-Nickel in Verbindung mit NH₃ durchgeführt wird.

5. Verfahren nach einem der Ansprüche 1 bis 4, dadurch gekennzeichnet, daß die Hydrierung in Gegenwart von Methanol als Verdünnungsmittel durchgeführt wird.

6. Verfahren nach Anspruch 1, dadurch gekennzeichnet, daß die Umsetzung von 3,5-Dimethyl-4-methoxypyridin-2-methanamin in Gegenwart von Wasser und Eisessig im Verhältnis von 9 : 1 bis 1 : 1 durchgeführt wird.

7. Verfahren nach einem der Ansprüche 1 bis 6, dadurch gekennzeichnet, daß die bei der Reaktion entstandenen Zwischenprodukte 3,5-Dimethyl-4-methoxypyridin-2methanamin und 3,5-Dimethyl-4-methoxypyridin-2-methanol ohne Isolierung zu einer Verbindung der allgemeinen Formel I, in der X den Rest Cl bedeutet, umgesetzt werden.

8. 3,5-Dimethyl-4-methoxypyridin-2-methanamin.

9. Verwendung von 3,5-Dimethyl-4-methoxypyridin-2-methanamin zur Herstellung von 3,5-Dimethyl-4-methoxypyridin-2-methanol und 3,5-Dimethyl-4-methoxy-2-chlormethylpyridin.

## Patentansprüche (Patentansprüche für folgende(n) Vertragsstaat(en): ES, GR)

1. Verfahren zur Herstellung von Verbindungen der allgemeinen Formel 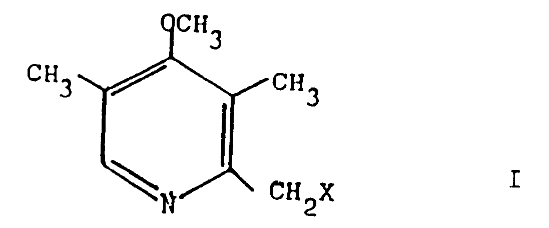 in der X die Reste OH oder Cl bedeutet, dadurch gekennzeichnet, daß man 3,5-Dimethyl-4-methoxypyridin-2-carbonitril der Formel 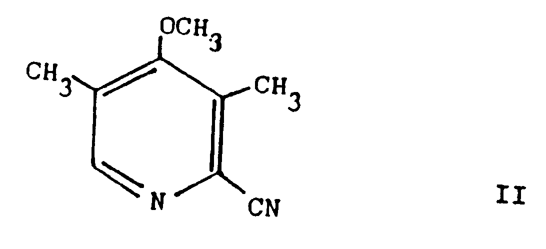 in Gegenwart eines inerten Verdünnungsmittels katalytisch hydriert, anschließend das entstandene 3,5-Dimethyl-4-methoxypyridin-2-methanamin der Formel 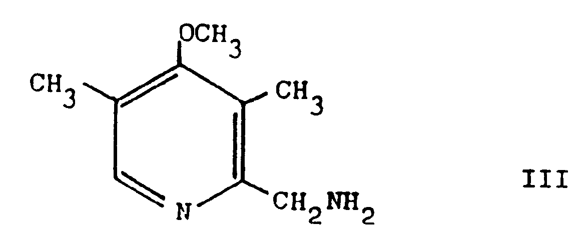 mit Natriumnitrit in wäßriger saurer Lösung zu 3,5-Dimethyl-4-methoxypyridin-2-methanol umsetzt und gegebenenfalls anschließend mit Thionylchlorid zu 3,5-Dimethyl-4-methoxy-2-chlormethylpyridin umsetzt.

2. Verfahren nach Anspruch 1, dadurch gekennzeichnet, daß die katalytische Hydrierung mit Raney-Nickel oder Pd auf Aktivkohle als Katalysator durchgeführt wird.

3. Verfahren nach einem der Ansprüche 1 oder 2, dadurch gekennzeichnet, daß die katalytische Hydrierung mit Raney-Nickel in Verbindung mit NH₃ oder mit Pd auf Aktivkohle in Verbindung mit Mineralsäuren oder Toluolsulfonsäure durchgeführt wird.

4. Verfahren nach einem der Ansprüche 1 bis 3, dadurch gekennzeichnet, daß die katalytische Hydrierung mit Raney-Nickel in Verbindung mit NH₃ durchgeführt wird.

5. Verfahren nach einem der Ansprüche 1 bis 4, dadurch gekennzeichnet, daß die Hydrierung in Gegenwart von Methanol als Verdünnungsmittel durchgeführt wird.

6. Verfahren nach Anspruch 1, dadurch gekennzeichnet, daß die Umsetzung von 3,5-Dimethyl-4-methoxypyridin-2-methanamin in Gegenwart von Wasser und Eisessig im Verhältnis von 9 : 1 bis 1 : 1 durchgeführt wird.

7. Verfahren nach einem der Ansprüche 1 bis 6, dadurch gekennzeichnet, daß die bei der Reaktion entstandenen Zwischenprodukte 3,5-Dimethyl-4-methoxypyridin-2methanamin und 3,5-Dimethyl-4-methoxypyridin-2-methanol ohne Isolierung zu einer Verbindung der allgemeinen Formel I, in der X den Rest Cl bedeutet, umgesetzt werden.

8. Verwendung von 3,5-Dimethyl-4-methoxypyridin-2-methanamin zur Herstellung von 3,5-Dimethyl-4-methoxypyridin-2-methanol und 3,5-Dimethyl-4-methoxy-2-chlormethylpyridin.

## Claims (Claims for the following Contracting State(s): AT, BE, CH, DE, FR, GB, IT, LI, LU, NL, SE)

1. Process for the preparation of compounds of the general formula 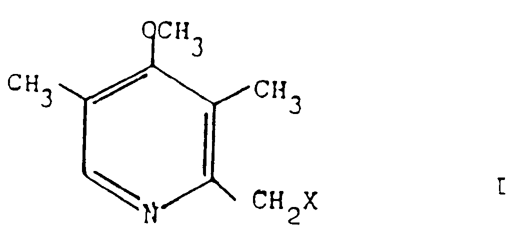 in which X is the radical OH or Cl, characterised in that 3,5-dimethyl-4-methoxy-2-cyanopyridine of the formula 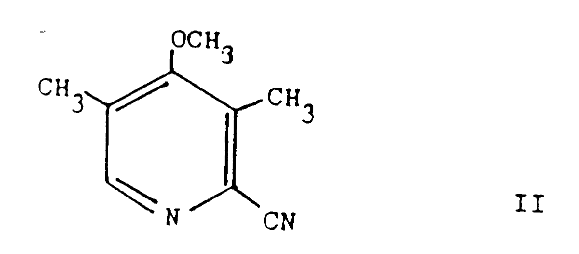 is catalytically hydrogenated in the presence of an inert diluent, the resulting 3,5-dimethyl-4-methoxy-2-aminomethylpyridine of the formula 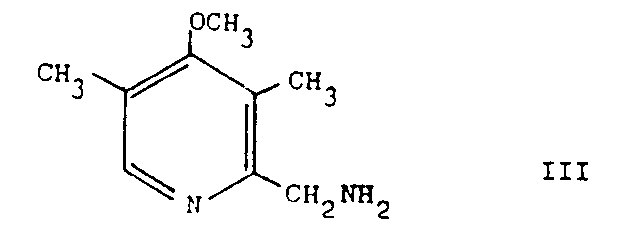 is then reacted with sodium nitrite in aqueous-acidic solution to give 3,5-dimethyl-4-methoxy-2-hydroxymethylpyridine and, if desired, the latter is then reacted with thionyl chloride to give 3,5-dimethyl-4-methoxy-2-chloromethylpyridine.

2. Process according to Claim 1, characterised in that the catalytic hydrogenation is carried out with Raney nickel or Pd on active charcoal as the catalyst.

3. Process according to Claim 1 or Claim 2, characterised in that the catalytic hydrogenation is carried out with Raney nickel in combination with NH₃ or with Pd on active charcoal in combination with mineral acids or toluene-sulphonic acid.

4. Process according to any one of Claims 1 to 3, characterised in that the catalytic hydrogenation is carried out with Raney nickel in combination with NH₃.

5. Process according to any one of Claims 1 to 4, characterised in that the hydrogenation is carried out in the presence of methanol as the diluent.

6. Process according to Claim 1, characterised in that the reaction of 3,5-dimethyl-4-methoxy-2-aminomethylpyridine is carried out in the presence of water and glacial acetic acid in a ratio of 9:1 to 1:1.

7. Process according to any one of Claims 1 to 6, characterised in that the intermediates formed in the reaction, 3,5-dimethyl-4-methoxy-2-aminomethylpyridine and 3,5-dimethyl-4-methoxy-2-hydroxymethylpyridine, are reacted, without isolation, to give a compound of general formula I in which X is the radical Cl.

8. 3,5-Dimethyl-4-methoxy-2-aminomethylpyridine.

9. Use of 3,5-dimethyl-4-methoxy-2-aminomethylpyridine for the preparation of 3,5-dimethyl-4-methoxy-2-hydroxymethylpyridine and 3,5-dimethyl-4-methoxy-2-chloromethylpyridine.

## Claims (Claims for the following Contracting State(s): ES, GR)

1. Process for the preparation of compounds of the general formula 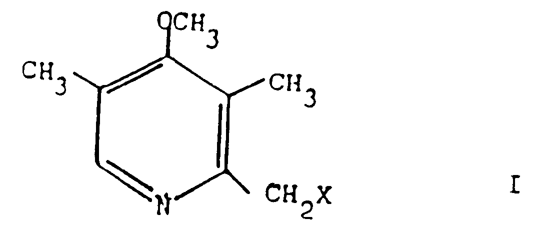 in which X is the radical OH or Cl, characterised in that 3,5-dimethyl-4-methoxy-2-cyanopyridine of the formula 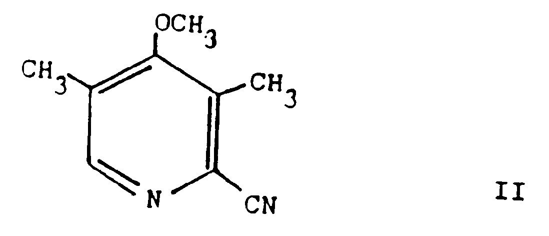 is catalytically hydrogenated in the presence of an inert diluent, the resulting 3,5-dimethyl-4-methoxy-2-aminomethylpyridine of the formula 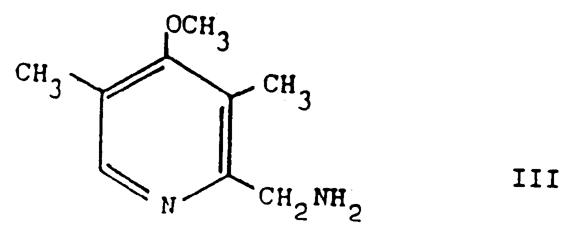 is then reacted with sodium nitrite in aqueous-acidic solution to give 3,5-dimethyl-4-methoxy-2-hydroxymethylpyridine and, if desired, the latter is then reacted with thionyl chloride to give 3,5-dimethyl-4-methoxy-2-chloromethylpyridine.

2. Process according to Claim 1, characterised in that the catalytic hydrogenation is carried out with Raney nickel or Pd on active charcoal as the catalyst.

3. Process according to Claim 1 or Claim 2, characterised in that the catalytic hydrogenation is carried out with Raney nickel in combination with NH₃ or with Pd on active charcoal in combination with mineral acids or toluene-sulphonic acid.

4. Process according to any one of Claims 1 to 3, characterised in that the catalytic hydrogenation is carried out with Raney nickel in combination with NH₃.

5. Process according to any one of Claims 1 to 4, characterised in that the hydrogenation is carried out in the presence of methanol as the diluent.

6. Process according to Claim 1, characterised in that the reaction of 3,5-dimethyl-4-methoxy-2-aminomethylpyridine is carried out in the presence of water and glacial acetic acid in a ratio of 9:1 to 1:1.

7. Process according to any one of Claims 1 to 6, characterised in that the intermediates formed in the reaction, 3,5-dimethyl-4-methoxy-2-aminomethylpyridine and 3,5-dimethyl-4-methoxy-2-hydroxymethylpyridine, are reacted, without isolation, to give a compound of general formula I in which X is the radical Cl.

8. Use of 3,5-dimethyl-4-methoxy-2-aminomethylpyridine for the preparation of 3,5-dimethyl-4-methoxy-2 -hydroxymethylpyridine and 3,5-dimethyl-4-methoxy-2-chloromethylpyridine.

## Revendications (Revendications pour l'(les) Etat(s) contractant(s) suivant(s): AT, BE, CH, DE, FR, GB, IT, LI, LU, NL, SE)

1. Procédé de préparation de composé de formule générale I : 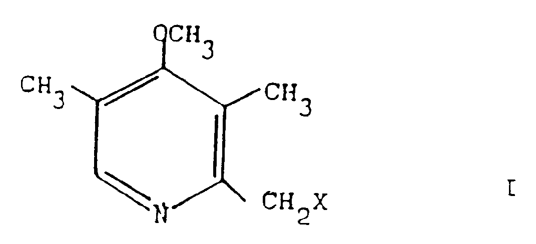 dans laquelle X désigne les restes OH ou Cl, caractérisé en ce qu'on hydrogénise la diméthyl-3,5-méthoxy-4-carbonitril-2-pyridine de formule II : 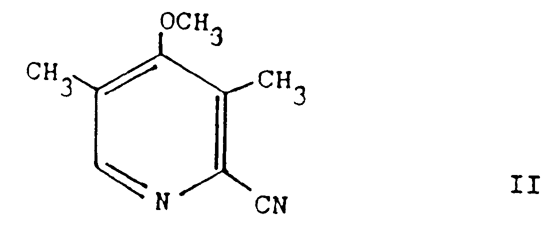 en présence d'un moyen de dilution inerte et d'un catalyseur on transforme ensuite la diméthyl-3,5-méthoxy-4-méthaneamino-2-pyridine obtenue de formule générale III : 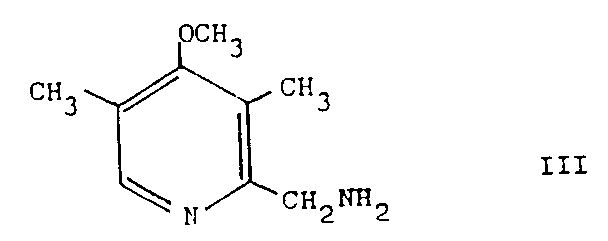 avec du nitrite de sodium en solution aqueuse acide en diméthyl-3,5-méthoxy-4-méthanol-2-pyridine qu'on transforme le cas échéant avec du chlorure de thionyle en diméthyl-3,5-méthoxy-4- chlorométhyl-2-pyridine.

2. Procédé selon la revendication 1, caractérisé en ce que l'hydrogénation catalytique est réalisée avec un nickel de Raney ou du palladium sur du charbon actif comme catalyseur.

3. Procédé selon l'une des revendications 1 ou 2, caractérisé en ce que l'hydrogénation catalytique avec le nickel de Raney est conduite en présence de NH₃ ou avec le palladium sur du charbon actif en présence d'acides minéraux ou d'acide toluolsulfonique.

4. Procédé selon l'une des revendications 1 à 3, caractérisé en ce que l'hydrogénation catalytique avec le nickel de Raney est conduite en présence de NH₃.

5. Procédé selon l'une des revendications 1 à 4,, caractérisé en ce que l'hydrogénation est conduite en présence de méthanol comme moyen de dilutione.

6. Procédé selon la revendication 1, caractérisé en ce que la réaction de la diméthyl-3,5-méthoxy-4-méthaneamino-2-pyridine est conduite en présence d'eau et d'acide acétique dans le rapport 9:1 à 1:1.

7. Procédé selon l'une des revendications 1 à 6, caractérisé en ce qu'on fait réagir les produits intermédiaires obtenus au cours de la réaction, la diméthyl-3,5-méthoxy-4-méthaneamino-2-pyridine et la diméthyl-3,5-méthoxy-4-méthanol-2-pyridine, sans les isoler en un composé de formule générale I dans laquelle X signifie le reste CI.

8. diméthyl-3,5-méthoxy-4-méthaneamino-2-pyridine.

9. Utilisation de la diméthyl-3,5-méthoxy-4-méthaneamino-2-pyridine à la préparation de la diméthyl-3,5-méthoxy-4-méthanol-2-pyridine et la diméthyl-3,5-méthoxy-4-chlorométhyl-2-pyridine.

## Revendications (Revendications pour l'(les) Etat(s) contractant(s) suivant(s): ES, GR)

1. Procédé de préparation de composé de formule générale I : 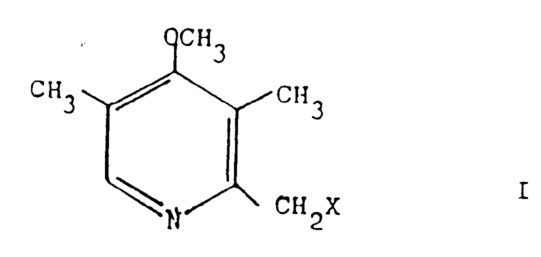 dans laquelle X désigne les restes OH ou Cl, caractérisé en ce qu'on hydrogénise la diméthyl-3,5-méthoxy-4-carbonitril-2-pyridine de formule II : 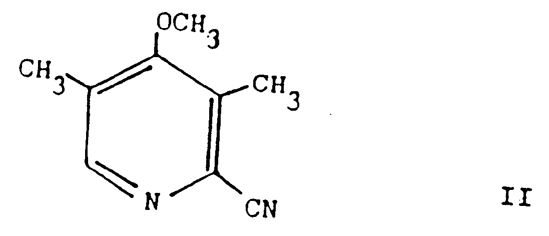 en présence d'un moyen de dilution inerte et d'un catalyseur on transforme ensuite la diméthyl-3,5-méthoxy-4-méthaneamino -2-pyridine obtenue de formule générale III : 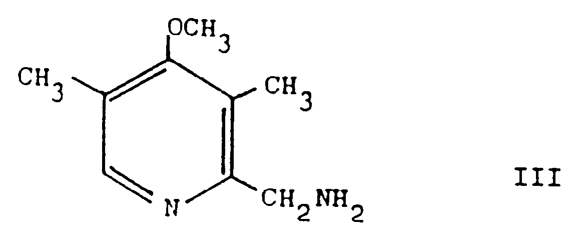 avec du nitrite de sodium en solution aqueuse en diméthyl-3,5-méthoxy-4-méthanol-2-pyridine qu'on transforme le cas échéant avec du chlorure de thionyle en diméthyl-3,5-méthoxy-4-chlorométhyl-2-pyridine.

2. Procédé selon la revendication 1, caractérisé en ce que l'hydrogénation catalytique est réalisée avec un nickel de Raney ou du palladium sur du charbon actif comme catalyseur.

3. Procédé selon l'une des revendications 1 ou 2, caractérisé en ce que l'hydrogénation catalytique avec le nickel de Raney est conduite en présence de NH₃ ou avec le palladium sur du charbon actif en présence d'acides minéraux ou d'acide toluolsulfonique.

4. Procédé selon l'une des revendications 1 à 3, caractérisé en ce que l'hydrogénation catalytique avec le nickel de Raney est conduite en présence de NH₃.

5. Procédé selon l'une des revendications 1 à 4,, caractérisé en ce que l'hydrogénation est conduite en présence de méthanol comme moyen de dilutione.

6. Procédé selon la revendication 1, caractérisé en ce que la réaction de la diméthyl-3,5-méthoxy-4-méthaneamino-2-pyridine est conduite en présence d'eau et d'acide acétique dans le rapport 9:1 à 1:1.

7. Procédé selon l'une des revendications 1 à 6, caractérisé en ce qu'on fait réagir les produits intermédiaires obtenus au cours de la réaction, la diméthyl-3,5-méthoxy-4-méthaneamino-2-pyridine et la diméthyl-3,5-méthoxy-4-méthanol-2-pyridine, sans les isoler en un composé de formule générale I dans laquelle X signifie le reste Cl.

8. Utilisation de la diméthyl-3,5-méthoxy-4-méthaneamino -2-pyridine à la préparation de la diméthyl-3,5-méthoxy-4-méthanol-2-pyridine et la diméthyl-3,5-méthoxy -4-chlorométhyl-2-pyridine.
